(19) Europäisches Patentamt
European Patent Office
Office européen des brevets

(11) Veröffentlichungsnummer: **0 034 741**
**B1**

(12) # EUROPÄISCHE PATENTSCHRIFT

(45) Veröffentlichungstag der Patentschrift: **21.12.83**

(21) Anmeldenummer: **81100879.6**

(22) Anmeldetag: **07.02.81**

(51) Int. Cl.³: **C 07 C 25/13**, C 07 C 47/55, C 07 C 49/807, C 07 C 49/813, C 07 C 63/70, C 07 C 79/12, C 07 C 121/52, C 07 B 9/00, C 07 C 17/14, C 07 C 45/43, C 07 C 45/63 // C07C51/363, C07C76/02, C07C120/00

(54) **Verfahren zur Herstellung eines substituierten Bromfluorbenzols und 3-Brom-4-fluorbenzonitril.**

(30) Priorität: **22.02.80 DE 3006685**

(43) Veröffentlichungstag der Anmeldung: **02.09.81 Patentblatt 81/35**

(45) Bekanntmachung des Hinweises auf die Patenterteilung: **21.12.83 Patentblatt 83/51**

(84) Benannte Vertragsstaaten: **CH DE FR GB LI NL**

(56) Entgegenhaltungen:
**EP - A - 0 017 882**
**EP - A - 0 024 516**
**EP - A - 0 024 624**
**DE - A - 2 225 695**
**DE - A - 2 550 261**
**FR - A - 835 727**
**US - A - 3 013 079**

**JOURNAL OF THE CHEMICAL SOCIETY, 1950, Seiten 573-577 D.H. DERBYSHIRE et al.: "The significance of the bromine cation in aromatic substitution. Part II. Preparative applicability"**
**RECUELL DES TRAVAUX CHIMIQUES DES PAYS BAS, Band 83, Nr. 9-10, September-Oktober 1964, Seiten 1142-1148 Den Haag, NL. N.N. QUANG et al.: "Orientation dans la réaction de Friedel-Crafts d'acétylation des bromofluoro-benzènes"**
**THE JOURNAL OF ORGANIC CHEMISTRY, Band 21, Nr. 9, 16. Oktober 1956, Seiten 934-938 J.F. BUNNETT et al.: "The von Richter Reaction. III. Substituent Effects"**
**COMPTES RENDUS HEBDOMADAIRES DES SEANCES DE L'ACADEMIE DES SCIENCES Band 263, Nr. 2, Serie C, Séance du 11 juillet 1966, Seiten 145-148 BUI-KHAC-DIEP: "Orientation dans les réactions d'aroylation de Friedel et Crafts des fluorobromobenzènes"**

(73) Patentinhaber: **RIEDEL-DE HAEN AKTIENGESELLSCHAFT, Wunstorfer Strasse 40, D-3016 Seelze 1 (DE)**

(72) Erfinder: **Eiglmeier, Kurt, Dr., Kattowitzer Weg 5, D-3057 Neustadt (DE)**
Erfinder: **Knieps, Reinhard, Lettow-Vorbeck-Allee 8, D-3000 Hannover (DE)**

(74) Vertreter: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

EP 0 034 741 B1

# Verfahren zur Herstellung eines substituierten Bromfluorbenzols und 3-Brom-4-fluorbenzonitril

Die Erfindung bezieht sich auf ein Verfahren zur Herstellung eines substituierten Bromfluorbenzols durch direkte Bromierung von substituiertem Fluorbenzol, und auf 3-Brom-4-fluorbenzonitril.

Die Herstellung bestimmter 3-Brom-4-fluorbenzolderivate ist bereits bekannt. So wird 3-Brom-4-fluorbenzoesäure durch Oxidation von 3-Brom-4-fluortoluol erhalten, das durch Diazotieren von 3-Brom-4-aminotoluol und kontrollierte Zersetzung des in Substanz isolierten Diazoniumtetrafluoroborats hergestellt wird; 3-Brom-4-fluoracetophenon wird aus schwer zugänglichen 2-Fluorbrombenzol durch Umsetzung mit Acetylchlorid erhalten, wobei 2-Fluorbrombenzol aus 2-Fluoranilin hergestellt werden muß (vgl. Rec. Trav. Chim. 83 (1964), 1142). Ferner ist 3-Brom-4-fluornitrobenzol aus 4-Fluornitrobenzol mit Hilfe stöchiometrischer Mengen an teurem Silbersalz herstellbar (vgl. J. Prg. Chem. 21 (1956), 934). Diese Bromierung soll nach einem Verfahren durchgeführt werden, das aus J. Chem. Soc. 1950, Seiten 573 bis 577 bekannt ist und die Bromierung von Benzol und substituierten Benzolen in Gegenwart von Silbersalzen betrifft.

Weiterhin ist bekannt, daß 3-Brom-4-fluorbenzoesäure durch Bromierung von 4-Fluorbenzoesäure erhalten wird, und zwar bei einer Reaktionstemperatur von 60 bis 70°C unter Anwendung von Jod, Eisen und insbesondere Chlorsulfonsäure als Katalysator, jedoch ohne Angabe der benötigten Katalysatormengen (vgl. französische Patentschrift 835 727).

Aufgabe der Erfindung ist die Bereitstellung von substituiertem Bromfluorbenzol, das in technisch einfacher Weise aus gut zugänglichem Ausgangsmaterial herstellbar ist.

Die Erfindung betrifft nun ein Verfahren zur Herstellung eines substituierten Bromfluorbenzols der Formel (1)

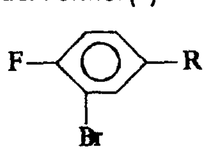

(1)

in der R einen Carboxylrest, einen Halogencarbonylrest, eine Nitrogruppe, eine Cyangruppe oder die Gruppe $-CO-R^1$ bedeutet, wobei $R^1$ ein Wasserstoffatom, einen niederen Alkylrest oder einen Arylrest darstellt, dadurch gekennzeichnet, daß ein substituiertes Fluorbenzol der Formel (2)

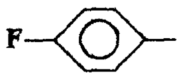

(2)

in der R die obengenannte Bedeutung hat, in Gegenwart von Jod in einer Menge von 0,02 bis 0,3 Mol oder eines dreiwertigen Metalls in einer Menge von 0,02 bis 0,3 Mol oder eines Halogenids eines dreiwertigen Metalls in einer Menge von 1 bis 1,2 Mol jeweils pro Mol des Fluorbenzols der Formel (2) einer direkten Bromierung mit Brom in einer Menge von 0,9 bis 1,3 Mol pro Mol des Fluorbenzols der Formel (2) bei einer Temperatur von 20 bis 200°C unterworfen wird.

Die Erfindung betrifft insbesondere ein substituiertes Bromfluorbenzol der Formel (3)

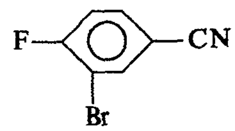

(3)

nämlich 3-Brom-4-fluorbenzonitril.

Als Ausgangsmaterial für das erfindungsgemäße Verfahren eignen sich insbesondere 4-Fluorbenzoylchlorid, 4-Fluorbenzoylbromid, 4-Fluorbenzaldehyd, 4-Fluoracetophenon, 4-Fluorbenzonitril, 4-Fluornitrobenzol, 4-Fluorbenzoesäure und 4-Fluorbenzophenon.

Das erfindungsgemäße Verfahren wird in Gegenwart eines Katalysators durchgeführt. Als Katalysator eignen sich Jod, dreiwertige Metalle, z. B. Eisen, und Halogenide von dreiwertigen Metallen, z. B. Eisen(III)-halogenide wie Eisen(III)-chlorid und Eisen(III)-bromid sowie Aluminium(III)-halogenide wie Aluminium(III)-chlorid und Aluminium(III)-bromid. Der Katalysator wird in einer Menge von 0,02 bis 0,3 Mol, bei Jod und Metallen und 1 bis 1,2 Mol bei dreiwertigen Metallhalogeniden jeweils pro Mol Fluorbenzolderivat eingesetzt.

Bei Verwendung eines dreiwertigen Metallhalogenides als Katalysator ist die Anwesenheit eines organischen Lösemittels empfehlenswert, insbesondere eines aliphatischen Halogenkohlenwasserstoffs, vorzugsweise eines Chlorkohlenwasserstoffs, mit 1 oder 2 Kohlenstoffatomen, z. B. Chloroform, Methylenchlorid, Tetrachlorkohlenstoff und 1,2-Dichlorethan.

Ein wesentliches Merkmal des erfindungsgemäßen Verfahrens ist die direkte Bromierung des Ausgangsmaterials. Hierzu wird elementares Brom in einer Menge von 0,9 bis 1,3 Mol, vorzugsweise 1,0 bis 1,2 Mol, pro Mol Fluorbenzolderivat verwendet. Vorteilhaft ist ein gleichzeitiger Zusatz von oxidierenden Agentien, vorzugsweise Chlor, wodurch der entstehende Bromwasserstoff zu Brom oxidiert wird, das ohne weiteres wieder für die Bromierung verwendet werden kann. Das oxidierende Agens wird gegebenenfalls in derselben molaren Menge wie das Brom eingesetzt; dadurch wird der Bedarf an Brom auf die Hälfte der an sich benötigten Menge vermindert.

Die Bromierung wird bei einer Temperatur von 20 bis 200°C durchgeführt. Bei Verwendung eines dreiwertigen Metalls als Katalysator ist eine Temperatur von 100 bis 170°C von Vorteil, während bei Gegenwart eines dreiwertigen Metallhalogenides als Katalysator eine Temperatur von 35 bis 60°C besonders angebracht ist.

Für die Herstellung von 3-Brom-4-fluorbenzaldehyd als substituiertes Bromfluorbenzol ist das erfindungsgemäße Verfahren in modifizierter Form besonders geeignet. Diese Variante besteht darin, daß in einer ersten Reaktionsstufe 4-Fluortoluol mit Brom zu 4-Fluorbenzalbromid umgesetzt wird, dieses dann in einer zweiten Reaktionsstufe zu 4-Fluorbenzaldehyd hydrolysiert und letzteres anschließend im Wege der direkten Bromierung in 3-Brom-4-fluorbenzaldehyd umgewandelt wird.

Dieses modifizierte Verfahren wird als Eintopfverfahren durchgeführt, da eine Isolierung der Zwischenstufen nicht erforderlich ist. Die Seitenkettenbromierung der ersten Reaktionsstufe wird vorzugsweise unter Einwirkung von kurzwelligem Licht oder in Gegenwart eines Katalysators durchgeführt; hierfür eignen sich Radikalbildner, insbesondere aliphatische Azoverbindungen, z. B. $\alpha,\alpha'$-Azoisobutyronitril, Azodicarbonsäurediamid, Azodicarbonsäuredialkylester wie Azodicarbonsäurediethylester, -diisopropylester oder -di-tert.butylester sowie Azo-$\gamma,\gamma'$-bis(4-cyanvaleriansäure). Auch die Hydrolyse der zweiten Reaktionsstufe wird vorzugsweise in Gegenwart eines Katalysators oder einer Base durchgeführt; der Katalysator ist vorzugsweise ein saures Salz wie Zinkchlorid und die Base eine anorganische Base wie Kaliumcarbonat oder Calciumcarbonat. Die Kernbromierung der dritten Reaktionsstufe erfolgt ebenfalls vorzugsweise in Gegenwart eines Katalysators, der insbesondere Jod, ein Metall oder ein Metallsalz ist, wie vorher beschrieben.

Die erfindungsgemäßen Verfahren sind in technisch einfacher und wirtschaftlicher Weise durchführbar. Die erhaltenen Bromfluorbenzolderivate weisen einen Reinheitsgrad von mehr als 95% vorzugsweise von 97 bis 99,5 Prozent, auf (ermittelt durch Gaschromatographie). Halogenisomerisierungen am aromatischen Kern des Ausgangsmaterials wurden nicht beobachtet.

Die erfindungsgemäß hergestellten 3-Brom-4-fluorbenzol-Derivate sind wertvolle Zwischenprodukte, die sich vor allem zur Synthese von pharmazeutischen und pflanzenschützenden Wirkstoffe eignen.

Die folgenden Beispiele erläutern die Erfindung. Prozentangaben beziehen sich auf das Gewicht.

Beispiel 1

In 1,9 kg 1,2-Dichlorethan werden zunächst 0,965 kg (7,24 Mol) Aluminiumchlorid und dann 0,8 kg (5,79 Mol) 4-Fluoracetophenon so eingetragen, daß die Temperatur des Gemisches 45°C nicht übersteigt. Unter Einhalten der Temperatur von 45°C werden 1,02 kg (6,38 Mol) Brom innerhalb von 4 Stunden unter Rühren zugetropft, und das Gemisch wird dann noch 3 Stunden nachgerührt. Anschließend wird das Gemisch in ein Gemisch aus 5,75 kg Eis und 0,535 kg Salzsäure (37prozentig) eingetragen. Nach Abtrennen der organischen Phase und Destillation wird 1 kg 3-Brom-4-fluoracetophenon erhalten (78% der Theorie) das einen Schmelzbereich von 53 bis 55,5°C, einen Siedebereich von 125 bis 126°C (bei einem Druck von 20 mbar) und einen Reinheitsgrad von 98,7% aufweist.

Beispiel 2

Analog Beispiel 1 wird aus 4-Fluorbenzaldehyd 3-Brom-4-fluorbenzaldehyd hergestellt, das einen Schmelzbereich von 27 bis 29°C, einen Siedebereich von 112 bis 115°C (bei einem Druck von 25 mbar) und einen Reinheitsgrad von 99,0% aufweist.

Beispiel 3

Analog Beispiel 1 wird aus 4-Fluorbenzonitril 3-Brom-4-fluorbenzonitril hergestellt, das einen Schmelzbereich von 53,8 bis 55,5°C einen Siedebereich von 134 bis 136°C (bei einem Druck von 44 mbar) und einen Reinheitsgrad von 99,7% aufweist.

Beispiel 4

250 g (1,77 Mol) 4-Fluornitrobenzol werden mit 3,0 g (0,054 Mol) Eisenpulver versetzt und auf eine Temperatur von 140°C erwärmt. Im Laufe von 9 Stunden werden dann 450 g (2,81 Mol) Brom zugetropft, wobei ferner insgesamt weitere 15 g (0,27 Mol) Eisenpulver portionsweise zugeführt werden. Anschließend wird das Gemisch noch 6 Stunden nachgerührt. Nach Filtration wird das Filtrat in 530 g heißem n-Hexan aufgenommen. Nach Erkalten der Lösung werden 220 g kristallines 3-Brom-4-fluornitrobenzol erhalten (55,8% der Theorie), das einen Schmelzbereich von 55,5 bis 56,5°C und einen Reinheitsgrad von 99,0% aufweist.

Beispiel 5

Analog Beispiel 4 wird aus 4-Fluorbenzoylchlorid ein Gemisch aus 3-Brom-4-fluorbenzoylchlorid und 3-Brom-4-fluorbenzoylbromid erhalten. Durch Destillation dieses Gemisches wird 3-Brom-4-fluorbenzoylbromid in Form farbloser Kristalle mit einem Schmelzbereich von 34,8 bis 35,8°C und einem Reinheitsgrad von 99,3% isoliert.

Beispiel 6

250 g (2,27 Mol) 4-Fluortoluol werden mit 1,25 g (7,61 mMol) $\alpha,\alpha'$-Azoisobutyronitril ver-

setzt und auf eine Temperatur von 110°C erwärmt. Im Laufe von 4 Stunden werden dann 725 g (4,54 Mol) Brom zugetropft, wobei die Temperatur des Reaktionsgemisches auf 145°C gesteigert wird. Das im wesentlichen aus 4-Fluorbenzalbromid bestehende Gemisch wird dann mit 1,25 g (9,17 mMol) Zinkchlorid versetzt, und danach werden innerhalb von 6 Stunden 40 g Wasser zudosiert. Nach Abkühlen auf eine Temperatur von 80°C und Zugabe von 1160 g (930 ml) 1,2-Dichlorethan wird das Gemisch auf 15°C abgekühlt. Dann werden 500 g Aluminiumchlorid portionsweise in das Gemisch eingetragen, wobei die Temperatur 20°C nicht übersteigt. Anschließend wird das Gemisch auf 40°C erwärmt, und im Laufe von 6 Stunden werden 382 g (2,39 Mol) Brom zugetropft; danach wird das Gemisch noch 16 Stunden lang bei einer Temperatur von 40°C nachgerührt. Das Reaktionsgemisch wird langsam auf ein Gemisch aus 2400 g Eis und 105 g konzentrierter Salzsäure gegossen und 5 Minuten lang gut gerührt. Die organische Phase des Gemisches wird abgetrennt und jeweils zweimal mit Wasser und 10prozentiger wäßriger Natriumcarbonatlösung sowie einmal mit Wasser gewaschen. Nach Abdampfen des 1,2-Dichlorethans wird das Produkt fraktioniert, destilliert. Man erhält 210 g (45%, bezogen auf 4-Fluortoluol) 3-Brom-4-fluorbenzaldehyd, das einen Siedebereich von 92 bis 98°C (bei einem Druck von 17 mbar) und einen Reinheitsgrad von >99% aufweist.

## Patentansprüche

1. Verfahren zur Herstellung eines substituierten Bromfluorbenzols der Formel (1)

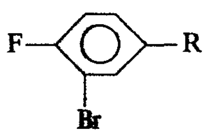 (1)

in der R einen Carboxylrest, einen Halogencarbonylrest, eine Nitrogruppe, eine Cyangruppe oder die Gruppe $-CO-R^1$ bedeutet, wobei $R^1$ ein Wasserstoffatom, einen niederen Alkylrest oder einen Arylrest darstellt, dadurch gekennzeichnet, daß ein substituiertes Fluorbenzol der Formel (2)

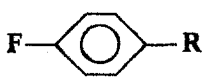 (2)

in der R die obengenannte Bedeutung hat, in Gegenwart von Jod in einer Menge von 0,02 bis 0,3 Mol oder eines dreiwertigen Metalls in einer Menge von 0,02 bis 0,3 Mol oder eines Halogenids eines dreiwertigen Metalls in einer Menge von 1 bis 1,2 Mol jeweils pro Mol des Fluorbenzols der Formel (2) einer direkten Bromierung mit Brom in einer Menge von 0,9 bis 1,3 Mol pro Mol des Fluorbenzols der Formel (2) bei einer Temperatur von 20 bis 200°C unterworfen wird.

2. Verfahren nach Anspruch 1, dadurch gekennzeichnet, daß die Bromierung in Gegenwart von Chlor durchgeführt wird, das in derselben molaren Menge wie das Brom eingesetzt wird.

3. Verfahren nach Anspruch 1, dadurch gekennzeichnet, daß als Katalysator ein dreiwertiges Metall eingesetzt und die Bromierung bei einer Temperatur von 100 bis 170°C durchgeführt wird.

4. Verfahren zur Herstellung von 3-Brom-4-fluorbenzaldehyd, dadurch gekennzeichnet, daß 4-Fluortoluol mit Brom zu 4-Fluorbenzalbromid umgesetzt wird, dieses dann zu 4-Fluorbenzaldehyd hydrolysiert wird und letzteres schließlich einer direkten Bromierung gemäß Anspruch 1 unterworfen wird.

5. Substituiertes Bromfluorbenzol der Formel (3)

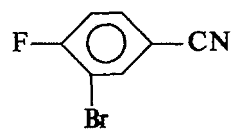

## Claims

1. Process for the manufacture of a substituted bromofluorobenzene of formula (1)

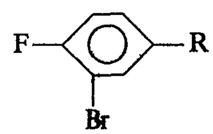 (1)

wherein R is a carboxy, halocarboxy, nitro, cyano group or a $-CO-R^1$ group with $R^1$ being a hydrogen atom, a lower alkyl or an aryl group, which comprises subjecting a substituted fluorobenzene of formula (2)

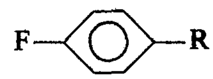 (2)

in which R is as defined before to a direct bromination with bromine in an amount of 0.9 to 1.3 mols per mol of the fluorobenzene of formula (2) at a temperature of 20 to 200°C in the presence of iodine in an amount of 0.02 to 0.3 mol or of a trivalent metal in an amount of 0.02 to 0.3 mol or of a halogenide of a trivalent metal in an amount of 1 to 1.2 mol per mol of the fluorobenzene of formula (2).

2. A process as claimed in claim 1, in which the bromination is carried out in the presence of chlorine used in the same molar amount as the bromine.

3. A process as claimed in claim 1, in which a trivalent metall is used as a catalyst and the bromination is carried out at a temperature of 100 to 170°C.

4. A process for the manufacture of 3-bromo-4-fluorobenzaldehyde, in which 4-fluorotoluene is reacted with bromine to 4-fluorobenzylbromide and this is hydrolyzed to 4-fluorobenzaldehyde which is subjected to direct bromination as claimed in claim 1.

5. Substituted bromofluorobenzene of formula (3)

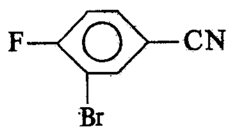

**Revendications**

1. Procédé de préparation d'un bromogluorobenzène substitué de formule (1)

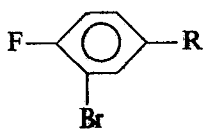 (1)

(dans laquelle R représente un radical carboxyle ou halogénocarbonyle, un groupe nitro ou cyano ou encore un groupe $-CO-R^1$, $R^1$ étant un atome d'hydrogène, un alkyle inférieur ou un radical aryle), procédé caractérisé en ce que l'on soumet un fluorobenzène substitué de formule (2)

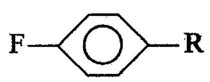 (2)

R ayant la signification ci-dessus, en présence d'iode à raison de 0,02 à 0,3 mole, ou bien d'un métal trivalent à raison de 0,02 à 0,3 mole également, ou encore d'un halogénure d'un métal trivalent à raison de 1 à 1,2 mole, dans tous les cas par mole du fluorobenzène de formule (2), à une bromation directe par le brome dans une proportion de 0,9 à 1,3 mole par mole du fluorobenzène de formule (2), à une température de 20 à 200° C.

2. Procédé selon la revendication 1 caractérisé en ce que la bromation se fait en présence de chlore, qui est ajouté dans la même proportion molaire que le brome.

3. Procédé selon la revendication 1 caractérisé en ce que l'on emploie comme catalyseur un métal trivalent et on effectue la bromation à une température de 100 à 170° C.

4. Procédé de préparation du 3-bromo-4-fluorobenzaldéhyde, caractérisé en ce que l'on fait réagir le 4-fluorotoluène avec du brome pour former le bromure de 4-fluorobenzylidène, que l'on hydrolyse ensuite en 4-fluorobenzaldéhyde, puis on soumet ce dernier à une bromation directe selon la revendication 1.

5. Un bromofluorobenzène substitué de formule